(19) Europäisches Patentamt
European Patent Office
Office européen des brevets

(11) Veröffentlichungsnummer: **0 087 656**
**B1**

(12) # EUROPÄISCHE PATENTSCHRIFT

(45) Veröffentlichungstag der Patentschrift: **07.05.86**

(51) Int. Cl.[4]: **C 07 D209/28**

(21) Anmeldenummer: **83101354.5**

(22) Anmeldetag: **12.02.83**

(54) **Verfahren zur Herstellung von 1-(4-Chlorbenzoyl)-5-methoxy-2-methyl-3-indolacetoxyessigsäure.**

(30) Priorität: **26.02.82 DE 3206888**

(43) Veröffentlichungstag der Anmeldung: **07.09.83 Patentblatt 83/36**

(45) Bekanntmachung des Hinweises auf die Patenterteilung: **07.05.86 Patentblatt 86/19**

(84) Benannte Vertragsstaaten: **AT BE CH DE FR GB IT LI LU NL SE**

(56) Entgegenhaltungen: **CHEMICAL ABSTRACTS, Band 90, Nr. 10, 5. März 1979, Seite 60, Nr. 87267x, Columbus, Ohio, USA**
**Die Akte enthält technische Angaben, die nach dem Eingang der Anmeldung eingereicht wurden und die nicht in dieser Patentschrift enthalten sind.**

(73) Patentinhaber: **Troponwerke GmbH & Co. KG**
**Berliner Strasse 156**
**D-5000 Köln 80 (DE)**

(72) Erfinder: **Boltze, Karl-Heinz, Dr.**
**Ackerstrasse 8**
**D-5231 Borod (DE)**

(74) Vertreter: **Jesse, Ralf-Rüdiger, Dr. et al**
**Bayer AG Konzernverwaltung RP Patentabteilung**
**D-5090 Leverkusen 1 Bayerwerk (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 087 656 B1

Jouve, 18, rue St-Denis, 75001 Paris, France

**Beschreibung**

Die vorliegende Erfindung betrifft ein neues chemisch eigenartiges und vorteilhaftes Verfahren zur Herstellung der bekannten 1-(4-Chlorbenzoyl)-5-methoxy-2-methyl-3-indolacetoxyessigsäure (nachstehend als I bezeichnet).

Für die Herstellung dieser bekannten Verbindung sind bereits eine Reihe von Verfahren bekannt geworden, vgl. z. B. DE-OS 2 234 651, DE-OS 2 257 867 und DE-OS 2 943 125.

Bei den bekannten Verfahren wurde die Carboxygruppe zunächst durch einen Benzylrest geschützt, so daß in einem letzten Reaktionsschritt eine katalytische Hydrierung des Benzylesters gemäß folgendem Reaktionsschema durchgeführt werden mußte.

Reaktionsschema

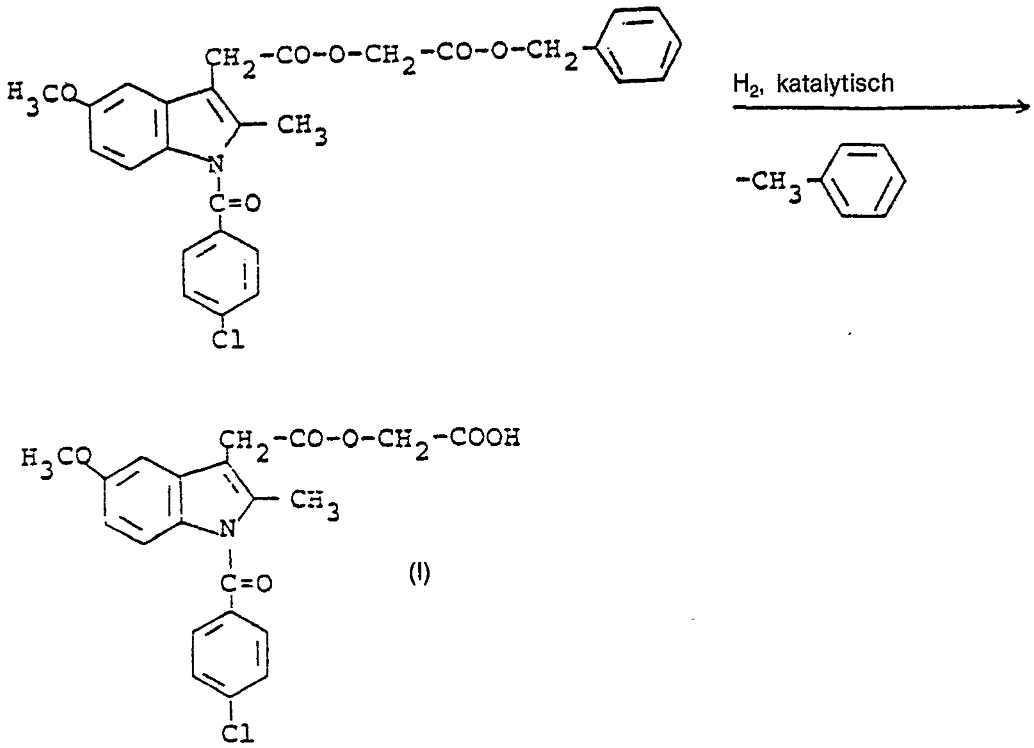


(I)

Bei dieser Abspaltung des Benzylrestes entsteht als Nebenprodukt stets die 1-Benzoyl-5-methoxy-2-methyl-3-indolacetoxyessigsäure, nachstehend Des-Chlor-Verbindung genannt. Diese unerwünschte Verunreinigung, die durch Abspaltung des Chlors aus dem Benzolring des 4-Chlorbenzoylrestes bis zu einer Menge von 0,5 % entsteht, muß in aufwendigen Reinigungsschritten anschließend entfernt werden, was mit Ausbeuteverlusten verbunden ist.

Aus C.A. 90, 87627x ist die Umsetzung von Indometacin mit Thionylchlorid in Ethylether/Pyridin zu einem Säurechlorid bekannt, das mit Glycolsäure Acemetacin ergibt.

Es wurde ein Verfahren zur Herstellung von 1-(4-Chlorbenzoyl)-5-methoxy-2-methyl-3-indolacetoxyessigsäure gefunden, das dadurch gekennzeichnet ist, daß man Derivate der Indolcarbonsäure der allgemeinen Formel

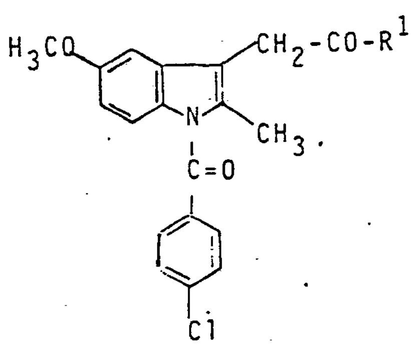

in welcher $R^1$ für einen der Reste

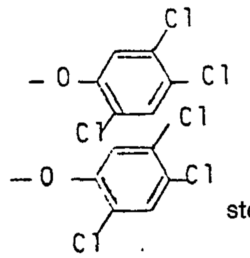

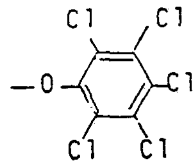

und

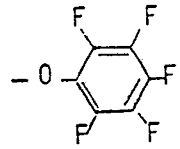

, und bevorzugt

steht,

mit Verbindungen der allgemeinen Formel

$$HO-CH_2-\overset{O}{\overset{\|}{C}}-O-R^2$$

in welcher $R^2$ für Wasserstoff oder Ammonium steht, in Gegenwart von inerten absoluten aprotischen organischen Lösungsmitteln in einem Temperaturbereich von —10 °C bis 80 °C umsetzt.

Verwendet man als Vertreter der allgemeinen Formel II die Indolcarbonsäurederivate, bei denen $R^1$ die obengenannten Substituenten bedeutet und Verbindungen der allgemeinen Formel III als Ausgangsstoffe, so kann der Reaktionsablauf durch das folgende Formelschema wiedergegeben werden :

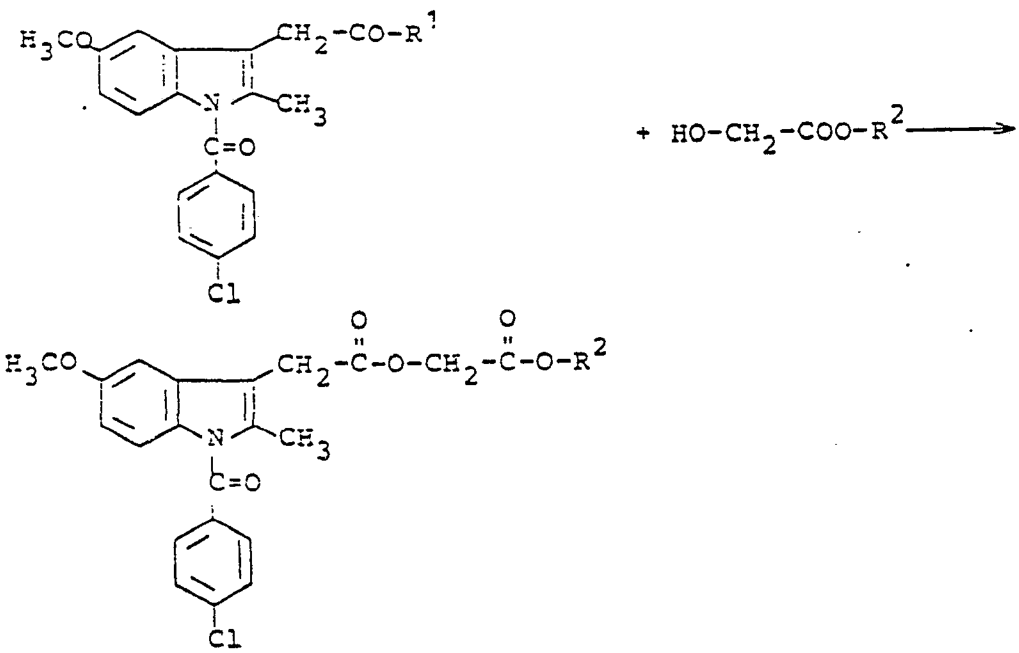

$R^1$ steht hier bevorzugt für

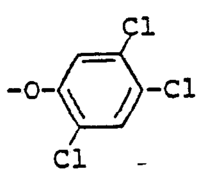

$R^2$ steht vorzugsweise für ein Ammoniumkation.

Die entstandenen Ammoniumverbindungen werden anschließend durch Behandlung mit Säuren, in einfacher Weise in das Endprodukt I überführt.

Es war überraschend, daß nach diesen Verfahren die 1-(4-Chlorbenzoyl)-5-methoxy-2-methyl-3-indolacetoxyessigsäure in reiner Form, d. h. frei von der störenden Des-Chlor-Verbindung, und in Ausbeuten von 60 bis 70 % der Theorie entsteht.

Die als Ausgangsstoffe verwendeten Verbindungen der Formel II und III sind bekannt oder werden nach bekannten Verfahren hergestellt.

Die nach den erfindungsgemäßen Verfahren hergestellte Endverbindung I ist ein wertvoller pharmazeutischer Wirkstoff mit antiphlogistischer Wirkung, vgl. z. B. DE-PS 2 234 651.

Im vorliegenden Verfahren wird die Indolcarbonsäure II ($R^1$ = OH) durch Esterbildung mit verschiedenen halogensubstituierten Phenolen funktionalisiert. Hierbei werden die 2,4,5-Trichlorphenyl-, 2,3,4,5,6-Pentachlorphenyl- und 2,3,4,5,6-Pentafluorphenylester verwendet, bevorzugt jedoch das 2,4,5-Trichlorphenylester. Die als Zwischenverbindungen hergestellten Ester sind sehr negativ gegenüber protonischen Reagenzien. Sie können unter bestimmten Bedingungen isoliert und rein dargestellt werden ; auch ist es möglich, sie als Aktivester herzustellen und sie im gleichen Ansatz sofort durch nukleophilen Angriff weiter umzusetzen.

Die feuchtigkeitsempfindlichen Aktivester verlangen Arbeiten in absoluten aprotischen Lösungsmitteln, wie Ether, z. B. Diethylether, Diisopropylether, Dioxan, Tetrahydrofuran, 1,2-Dimethoxyethan, chlorierten Kohlenwasserstoffen, z. B. Methylenchlorid, Chloroform, Dichlorethan, substituiertes Amiden, z. B. Dimethylformamid, N-Methyl-pyrrolidon, Aromaten, z. B. Toluol, Xylol, Ketonen, z. B. Aceton, Methylethylketon (Butanon-2-). Bevorzugt sind Methylenchlorid und Dimethylformamid.

Werden Indolcarbonsäure und Phenole in freier Form zur Umsetzung gebracht, ist die Verwendung eines Kondensationsmittels, hier beispielsweise Dicyclohexylcarbodiimid, erforderlich ; auch kann das Säurechlorid der Indolcarbonsäure mit oder ohne einer Base, beispielsweise Triethylamin, Pyridin oder Kaliumcarbonat sowie Natriumcarbonat, eingesetzt werden.

Bei der Umsetzung der Aktivester mit der bifunktionellen Glykolsäure entstehen viele Verbindungen, die schwer zu trennen sind. Erst die Anwendung von Ammoniumsalzen der Glykolsäure, insbesondere deren Triethyl- oder Diisopropylammoniumsalz führen zu guten Ausbeuten und hoher Reinheit der gewünschten Verbindungen.

Ein weiterer Vorteil dieser Salze von I, insbesondere der Diisopropylamminiumsalzes, liegt in der Möglichkeit, über sie die Endreinigung bis zu dem hohen Standard durchzuführen, der von einem pharmazeutischen Produkt gefordert wird.

Die Rekationstemperaturen liegen zwischen —10 und + 80 °C, bevorzugt zwischen 20 °C und 60 °C.

Zur Überführung der gereinigten Ammoniumsalze in die freie Indolcarbonsäure I schließt sich eine Behandlung mit Salzsäure an, wobei die Reaktionen und Kristallisation so durchgeführt werden, daß das Hydrat von I als farbloses Kristallisat in definierter Form erhalten wird. Die nachfolgende Trocknung im Vakuum bei 90° liefert unter vollständigem Wasserentzug gelbliche Kristalle von I. In analoger Reaktionsführung wird I aus den 2,3,4,5,6-Pentachlor- und 2,3,4,5,6-Pentafluorphenylestern von II erhalten.

Reaktionsschema : Synthese von I nach dem Aktivester-Verfahren

(Stufen a-d)

a)

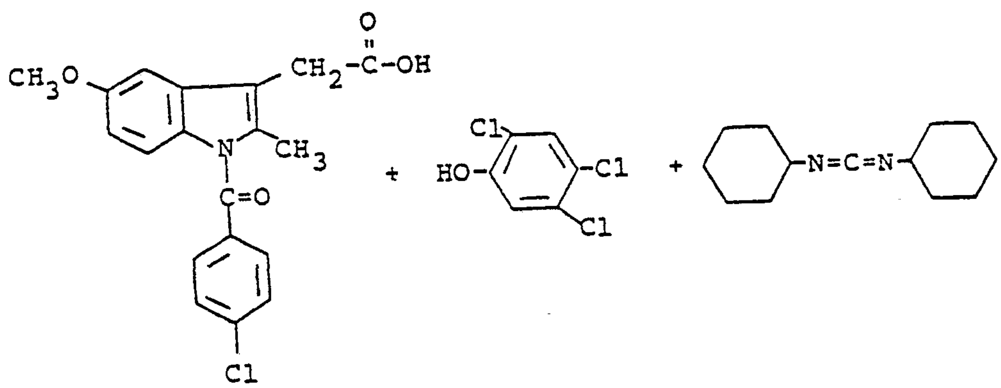

b)

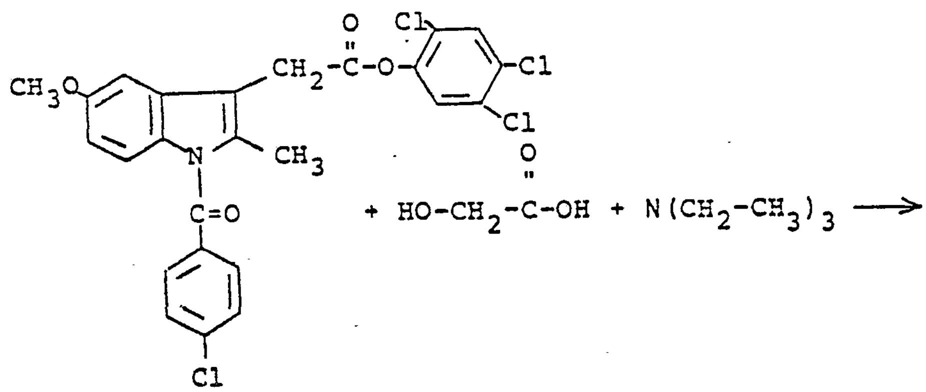

c)

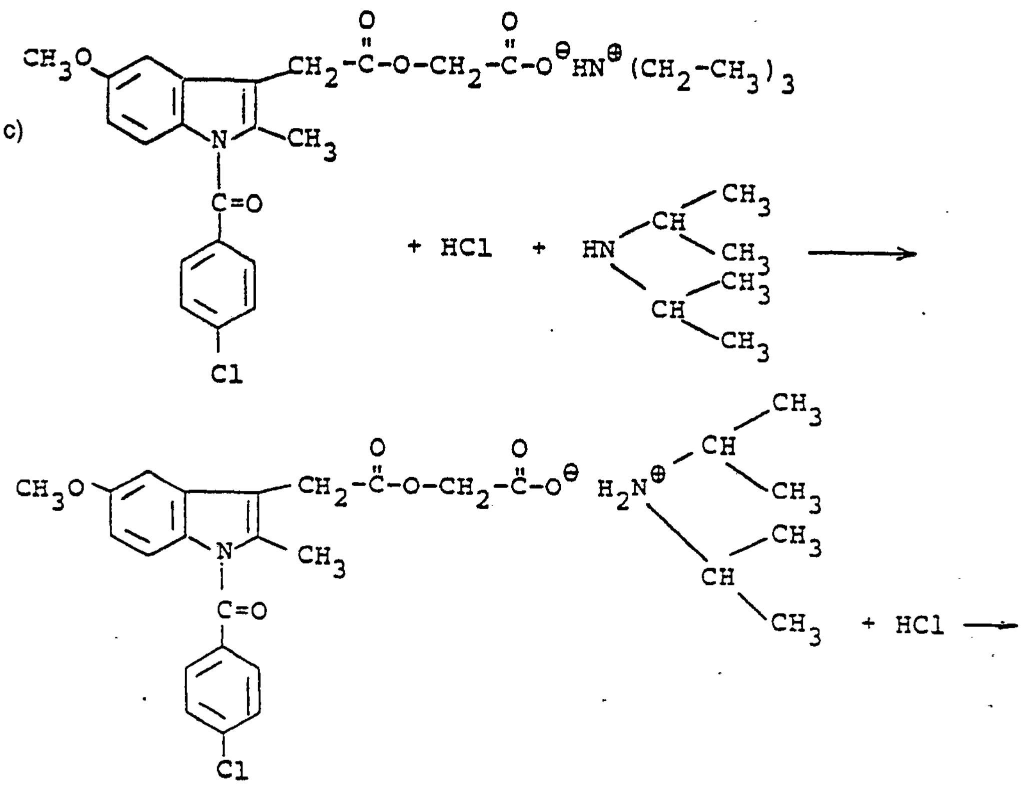

d)

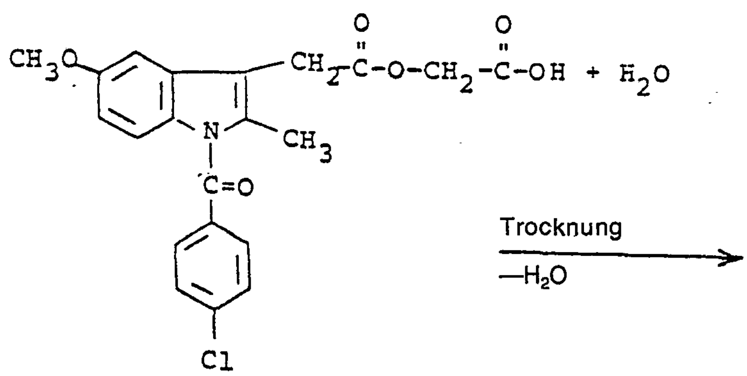


Beispiel 1

a) 2,4,5-Trichlorphenylester II,

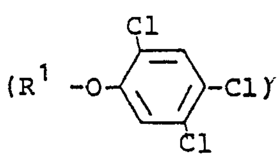

Zu einer auf —10 °C abegekühlten Lösung von 43,2 g II ($R^1$ OH) und 26,04 g 2,4,5-Trichlorphenol (0, 132 Mol) in 700 ml abs. Methylenchlorid werden unter Rühren und Feuchtigkeitsausschluß 24,8 g Dicyclohexylcarbodiimid (0,12 Mol) gegeben. Nach dem Entfernen des Kühlbades läßt man die Temperatur der Reaktionsmischung innerhalb 1 h auf Raumtemperatur ansteigen und filtriert den ausgefallenen farblosen kristallinen Feststoff ab. Das Filtrat wird im Rotationsverdampfer unter Wasserstrahlvakuum vom $CH_2Cl_2$ befreit und der gelbe sirupöse Rückstand mit 400 ml Petrolether versetzt. Das durch Animpfen und Reiben entstandene Kristallisat wird abgesaugt, mit Petrolether gewaschen und im Exsikkator unter Wasserstrahlvakuum getrocknet.

Ausbeute : 64,4 g 2,4,5-Trichlorphenyl-1-(4-Chlorbenzoyl)-5-methoxy-2-methyl-3-indolacetoxyacetat = 100 % d. Th. ; Fp. : 126,2 °C (Mettler FP 61).

In analoger Verfahrensweise werden hergestellt : 2,3,4,5,6-Pentachlorphenyl-1-(4-chlorbenzoyl)-5-methoxy-2-methyl-3-indolacetoxyacetat mit einem Fp. : 161,7 °C (Mettler FP 61) in einer Ausbeute von 85 % d. Th. und

2,3,4,5,6-Pentafluorphenyl-1-(4-chlorbenzoyl)-5-methoxy-2-methyl-3-indolacetoxyacetat mit einem Fp. : 131,6 °C (Mettler FP 61) in einer Ausbeute von ca. 100 % d. Th. ; (vgl. Lit. : J. Pless, R.A. Boissonnas, Helvitica *46*, 1609 (1963).

b) Umsetzung mit Triethylammoniumglykolat

c) Umwandlung in das Diisopropylammoniumsalz von I.

Zu einer Lösung von 10,74 g II

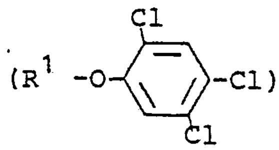

(0,02 Mol) und 2,28 g Glykolsäure (0,03 Mol) in 50 ml abs. Dimethylformamid (DMF) werden unter Rühren und Feuchtigkeitsausschluß bei Raumtemperatur 4,15 ml Triethylamin (0,03 Mol) zugetropft und noch 18 h lang weitergerührt. Anschließend erwärmt man die Reaktionslösung 2 1/2 h auf 60 °C und destilliert dann das Lösungsmittel im Rotationsverdampfer unter Wasserstrahlvakuum ab ; danach versetzt man den sirupösen Rückstand mit 80 ml Methylenchlorid. Die Methylenchloridlösung wird mit 25 ml 1 n HCl versetzt und Anschließend dreimal mit je 50 ml $H_2O$ gewaschen. Nach dem Trocknen der Methylenchloridphase mit $Na_2SO_4$ wird die Lösung mit 2,8 ml Diisopropylamin (0,02 Mol) versetzt und im Rotationsverdampfer $CH_2Cl_2$ abdestilliert. Der Rückstand wird in 80 ml Ether aufgenommen, und nach Animpfen und Rühren (1 h bei Raumtemperatur) wird die kristalline farblose Substanz abgesaugt, mit Ether gewaschen und bei 40 °C im Exsikkator getrocknet.

Ausbeute : 8,2 g Diisopropylammonium-1-(4-chlorbenzoyl)-5-methoxy-2-methyl-3-indolacetoxyacetat

d) Überführung in I

8,2 g des so gewonnenen Ammoniumsalzes werden unter Rühren in 32 ml Aceton und 10,5 ml $H_2O$ gelöst. Anschließend gibt man 6 ml 1 n HCl zu dieser Lösung und impft an. Innerhalb 1 h tropft man 10 ml 1 n HCl zu und rührt 1 h nach, bis die Kristallisation vollständig ist. Die farblose kristalline Substanz wird abgesaugt, mit $H_2O$ gut gewaschen und im Exsikkator bei 40 °C im Wasserstrahlvakuum getrocknet.

Ausbeute : Ausbeute 5,3 g I-Hydrat = 61 % d. Th. ;

Durch Trocknung bei 90 °C im Wasserstrahlvakuum (1 h) entstehen unter vollständigem Wasserentzug gelbliche Kristalle von I, die bei 151-152 °C schmelzen.

In analoger Verfahrensweise wird I hergestellt über die Synthesestufen b-d, sowohl aus dem Aktivester II

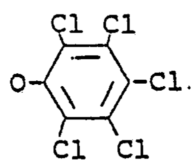

mit einer Ausbeute von 42 %
d. Th. als auch aus dem Aktivester II

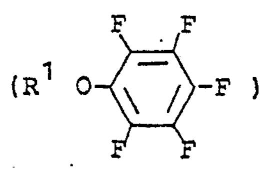

mit einer Ausbeute von 55 % d. Th.

**Patentansprüche**

1. Verfahren zur Herstellung von 1-(4-Chlorbenzoyl)-5-methoxy-2-methyl-3-indolacetoxyessigsäure,

dadurch gekennzeichnet, daß man Derivate der Indolcarbonsäure der allgemeinen Formel

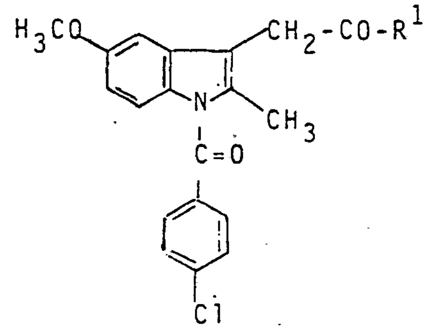

in welcher $R^1$ für einen der Reste

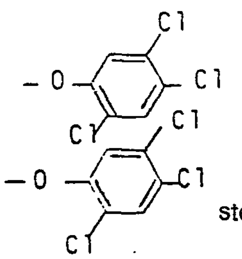

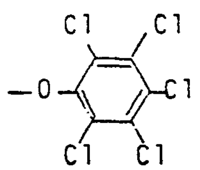

und

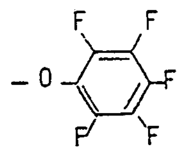

, und bevorzugt

steht,

mit Verbindungen der allgemeinen Formel

$$HO-CH_2-\overset{O}{\overset{\|}{C}}-O-R^2$$

in welcher $R^2$ für Wasserstoff oder Ammonium steht, in Gegenwart von inerten absoluten aprotischen organischen Lösungsmitteln in einem Temperaturbereich von —10 °C bis 80 °C umsetzt.

2. Verfahren nach Anspruch 1, dadurch gekennzeichnet, daß die Umsetzung in einem Temperaturbereich von —10 °C bis 50 °C vorgenommen wird.

3. Verfahren nach Anspruch 1 oder 2, dadurch gekennzeichnet, daß als inerte organische Lösungsmittel Ether, chlorierte Kohlenwasserstoffe, substituierte Amide, Aromaten und/oder Ketone, eingesetzt werden.

4. Verfahren nach einem oder mehreren der Ansprüche 1 bis 3, dadurch gekennzeichnet, daß als Derivat der Indolcarbonsäure die Verbindung

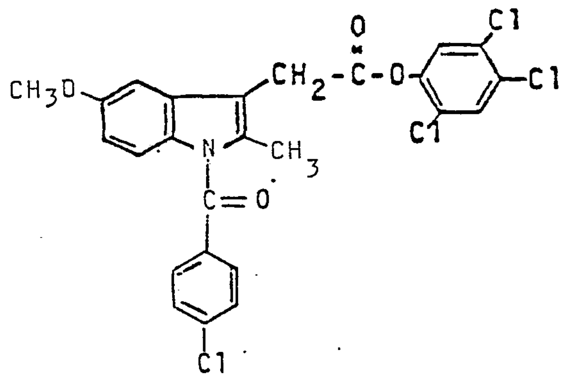

eingesetzt wird.

5. Verfahren nach einem oder mehreren der Ansprüche 1 bis 3, dadurch gekennzeichnet, daß das Ammoniumsalz der Glykolcarbonsäure eingesetzt wird.

**Claims**

1. Process for the preparation of 1-(4-chlorobenzoyl)-5-methoxy-2-methyl-3-indoleacetoxyacetic acid, characterised in that derivatives of indolecarboxylic acid of the general formula

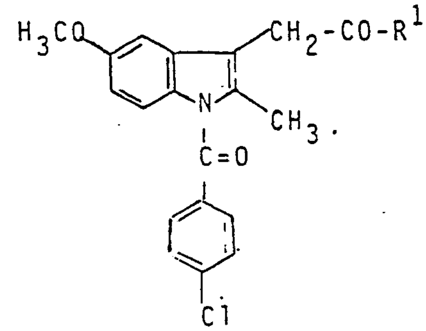

in which $R^1$ represents one of the radicals

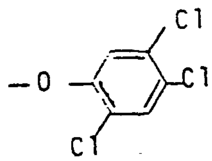 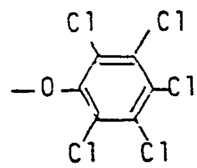 and 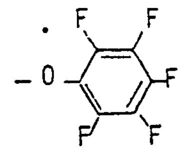,

and preferably 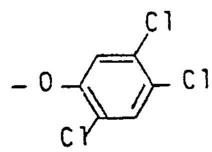

are reacted with compounds of the general formula

$$HO-CH_2-\overset{\overset{O}{\|}}{C}-O-R^2$$

in which $R^2$ represents hydrogen or ammonium, in the presence of inert, absolute, aprotic organic solvents, in a temperature range of —10 °C to 80 °C.

2. Process according to Claim 1, characterised in that the reaction is carried out in a temperature range of —10 °C to 50 °C.

3. Process according to Claim 1 or 2, characterised in that ethers, chlorinated hydrocarbons, substituted amides, aromatics and/or ketones are used as the inert organic solvents.

4. Process according to one or more of Claims 1 to 3, characterised in that the compound

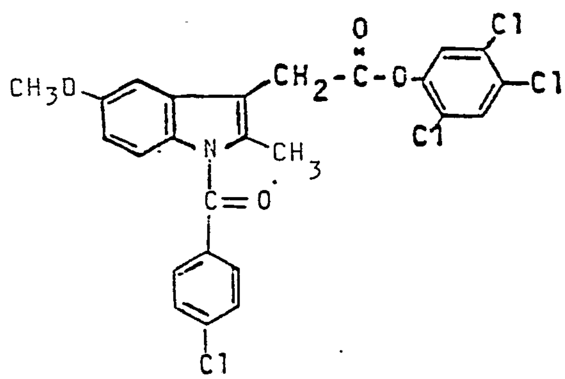

is used as the derivative of indolecarboxylic acid.

5. Process according to one or more of Claims 1 to 3, characterised in that the ammonium salt of glycol carboxylic acid is used.

**Revendications**

1. Procédé de production d'acide 1-(4-chlorobenzoyl)-5-méthoxy-2-méthyl-3-indolacétoxyacétique, caractérisé en ce qu'on fait réagir des dérivés de l'acide indolcarboxylique de formule générale

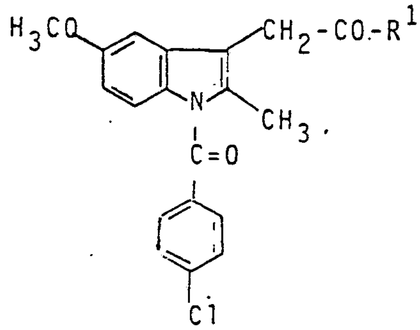

dans laquelle $R^1$ représente l'un des restes

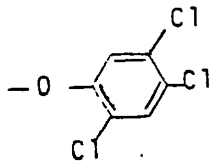

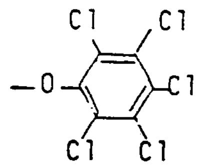

et

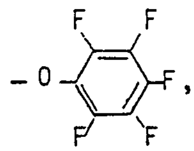

,

et de préférence

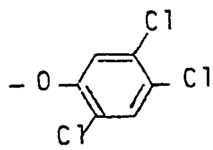

avec des composés de formule

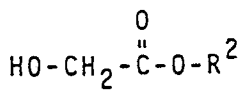

dans laquelle $R^2$ représente l'hydrogène ou l'ion ammonium en présence de solvants organiques aprotiques absolus inertes dans un intervalle de température de —10 °C à 80 °C.

2. Procédé suivant la revendication 1, caractérisé en ce que la réaction est conduite dans un intervalle de température de —10 °C à 50 °C.

3. Procédé suivant la revendication 1 ou 2, caractérisé en ce qu'on utilise comme solvants organiques inertes des éthers, des hydrocarbures chlorés, des amides substitués, des hydrocarbures aromatiques et/ou des cétones.

4. Procédé suivant une ou plusieurs des revendications 1 à 3, caractérisé en ce qu'on utilise comme dérivé de l'acide indolcarboxylique, le composé

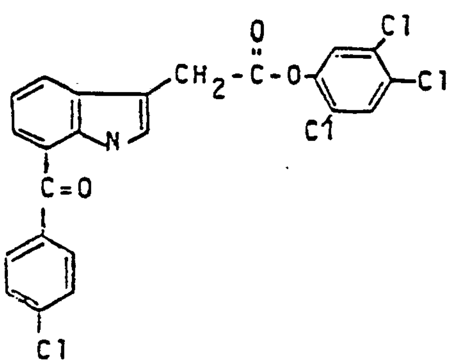

5. Procédé suivant une ou plusieurs des revendications 1 à 3, caractérisé en ce qu'on utilise le sel d'ammonium de l'acide glycolcarboxylique.